## Europäisches Patentamt
## European Patent Office
## Office européen des brevets

(19)

(11) Veröffentlichungsnummer: **0 002 803**
**B1**

(12) **EUROPÄISCHE PATENTSCHRIFT**

(45) Veröffentlichungstag der Patentschrift:
**01.04.81**

(51) Int. Cl.³: **A 61 K 31/045**

(21) Anmeldenummer: **78101797.5**

(22) Anmeldetag: **20.12.78**

(54) **Cis-(3,3,5)-Trimethylcyclohexanol zur Verwendung bei der Behandlung von Galleleiden.**

(30) Priorität: **23.12.77 DE 2757641**

(43) Veröffentlichungstag der Anmeldung:
**11.07.79 Patentblatt 79/14**

(45) Bekanntmachung des Hinweises auf die Patenterteilung:
**01.04.81 Patentblatt 81/13**

(84) Benannte Vertragsstaaten:
**BE CH DE FR GB IT LU NL SE**

(56) Entgegenhaltungen:
**VIDAL, 1967, O.V.P.,
Paris, FR,
«Rowacol»
Seite 1158**

(73) Patentinhaber: **ROWA LIMITED, Newtown, Bantry Cork (IE)**

(72) Erfinder: **Pailer, Mathias, Dr., Währinger Strasse 10, A-1040 Wien (AT)**
Erfinder: **Wagner, Roland, Am Mühlenweg 12, D-5065 Bergisch-Gladbach 1 (DE)**

(74) Vertreter: **Hassler, Werner, Dr. et al, Postfach 1704, D-5880 Lüdenscheid (DE)**

ACTORUM AG.

## Cis-(3,3,5)-Trimethylcyclohexanol zur Verwendung bei der Behandlung von Galleleiden

Aufgabe der Erfindung ist die Bereitstellung eines Arzneimittels mit hoher choleretischer Wirkung und guter Verträglichkeit bei der Behandlung von Galleleiden.

Cis-(3,3,5)-Trimethylcyclohexanol ist als Arzneimittel nicht bekannt.

Es hat sich überraschenderweise gezeigt, dass das cis-(3,3,5)-Trimethylcyclohexanol eine hohe Wirksamkeit für die Gallebildung hat. Daher ist diese Substanz in hohem Masse zur Behandlung von Galleleiden geeignet.

Gegenstand der Erfindung ist die Verwendung von cis-(3,3,5)-Trimethylcyclohexanol bei der Behandlung von Galleleiden und/oder bei der Erniedrigung der Cholesterinbildung in der Galle.

Die therapeutische Anwendung kann in Form von Tabletten, Kapseln, Dragees, Suppositorien, Rektalkapseln oder auch als Injektionslösung erfolgen.

Zum Nachweis der therapeutischen Wirksamkeit von cis-(3,3,5)-Trimethylcyclohexanol wurden Vorversuche an Ratten durchgeführt. Die Substanz wurde per oral appliziert, und die Veränderung des Galleflusses wurde gemessen. Die Untersuchungen sind in Anlehnung an das Verfahren von Boucard (vgl. Therapie 21, S. 903, 1966) durchgeführt. Nach der Präparation des Gallengangs wurde die gebildete Galle alle 30 Minuten gemessen. Jeweils nach 60 Minuten wurde die Substanz per oral appliziert. In weiteren Abständen von 30 Minuten wurde bei einer Gesamtzeit von 3 Stunden die Menge der gebildeten Galle gemessen. Die Messungen wurden mit einer Gruppe von Kontrolltieren, denen Erdnussöl appliziert wurde, einer Gruppe, denen eine Vergleichssubstanz appliziert wurde, und drei Gruppen, denen die erfindungsgemässe Substanz in unterschiedlicher Dosierung appliziert wurde, durchgeführt. Jeweils ist das Volumen des Galleflusses, der Cholesteringehalt und das Trockengewicht der Galleflüssigkeit bestimmt worden.

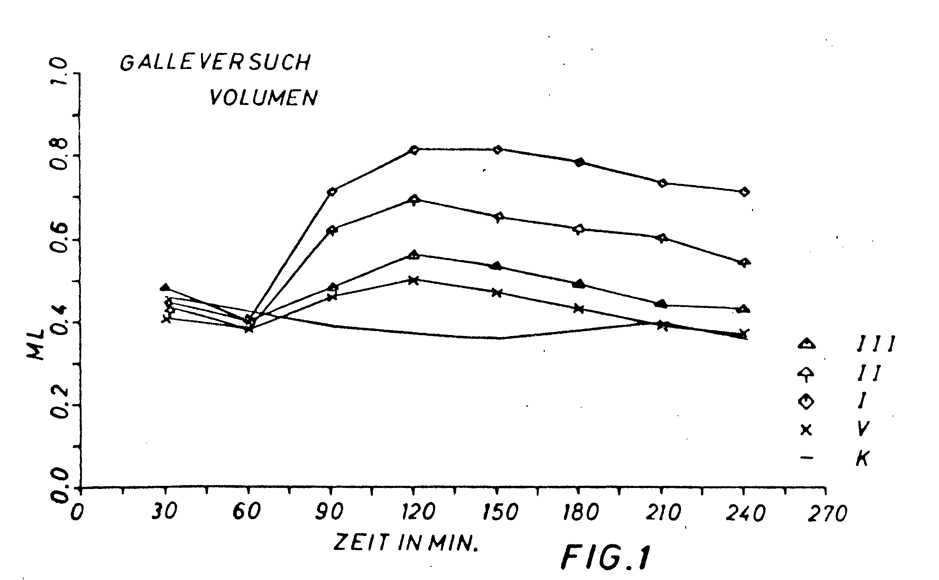

Fig. 1 zeigt die Messwerte für das Volumen des Galleflusses. Es sind die Messwerte für eine Kontrollgruppe K, eine Vergleichsgruppe V sowie drei Versuchsgruppen I, II, III angegeben, denen die Substanz nach der Erfindung in abnehmender Dosierung appliziert worden ist. Die Zeitskala beginnt mit dem Zeitpunkt der Präparation des Gallenganges. Jeweils nach 30 Minuten und nach 60 Minuten wurde der Gallefluss gemessen. Nach 60 Minuten wurde die jeweilige Substanz appliziert. Danach wurden 3 Stunden lang in Abständen von 30 Minuten die Messungen durchgeführt.

In den Versuchsgruppen I und II (entsprechend einer Dosierung von 0,3 ml/kg bzw. 0,15 ml/kg cis-(3,3,5)-Trimethylcyclohexanol) war im Vergleich zu der Kontrollgruppe eine hochgradige Erhöhung des Gallevolumens festzustellen. Die Signifikanz der Messwerte wurde nach dem Verfahren der Varianzanalyse unter Anwendung des Tuckey-Testes überprüft. Für die Substanz nach der Erfindung zeigt sich eine optimale Wirkung bei 60 und 90 Minuten nach der Applikation.

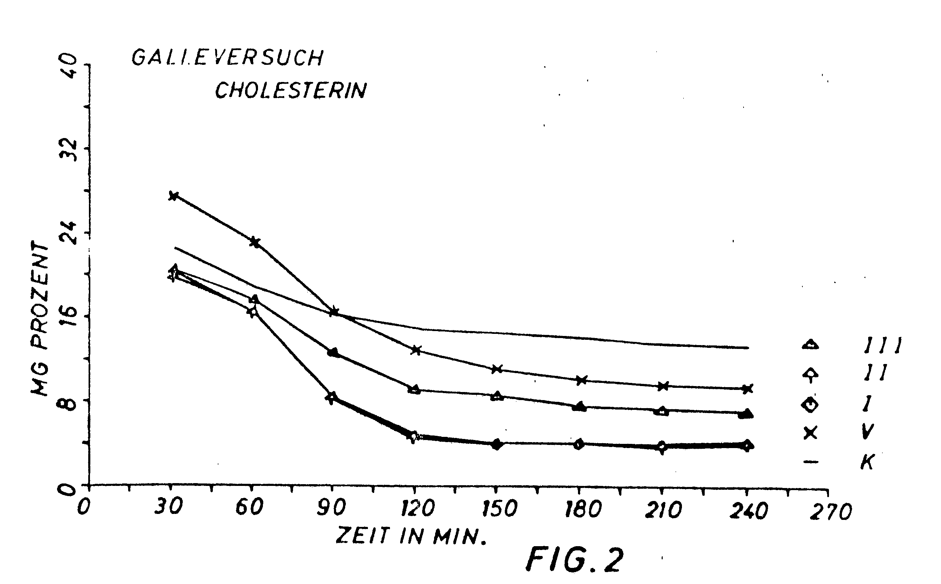

Fig. 2 gibt entsprechende Messungen für die Cholesterinwerte wieder. Die Versuchsgruppen I und II zeigen eine hochsignifikante Erniedrigung der Cholesterinwerte sowohl gegenüber der Kontrollgruppe K als auch gegenüber der Vergleichsgruppe V.

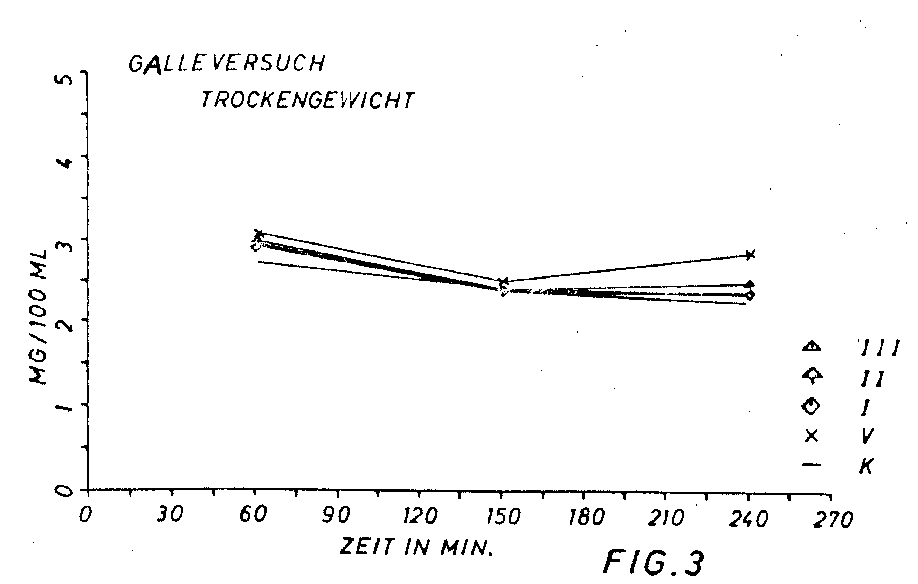

Schliesslich gibt Fig. 3 die Messwerte für das Trockengewicht an. Hier zeigen sich keine wesentlichen Unterschiede für die Messgruppen und die Kontrollgruppe.

Im Vergleich mit anderen klinisch erprobten Choleretika zeigt cis-(3,3,5)-Trimethylcyclohexanol die im folgenden geschilderte Wirkungspotenz. Die nachfolgend näher benannten Prüfsubstanzen wurden an männlichen Wistar-Ratten hinsichtlich ihrer choleretischen Wirkung überprüft; Ratten in Barbituratnarkose, Drainage des Ductus hepaticus und Sammeln der Galleflüssigkeit in Fraktionen alle 30 Minuten; Verabreichung der Prüfsubstanzen per os, nach einstündiger Vorperiode, Bezug: korrigierte Basissekretion zu den mit Leerpräparation behandelten Kontrollen.

Prüfsubstanzen:
Rowachol®, Terpengemisch aus 6 Einzelterpenen, Dosis 4,0 ml/kg
Decholin®, Dehydrocholsäure, Dosis 600 mg/kg
Felogen®, Bernsteinsäure-mono-$\alpha$-(2,5-endomethylen-$\Delta^3$-cyclohexenyl)-äthylester, Dosis 100 mg/kg
cis-(3,3,5)-Trimethylcyclohexanol, Dosis 0,3 ml/kg.

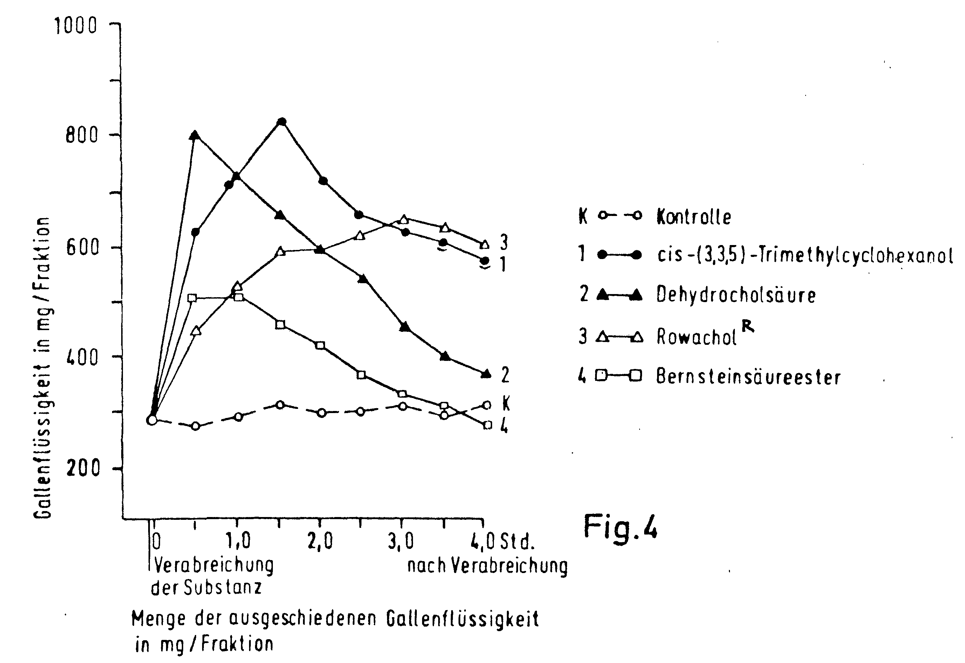

Die Versuchsergebnisse sind in Fig. 4 graphisch dargestellt.

Der Bernsteinsäureester führte zu einem deutlichen Anstieg der Cholerese mit einem Maximum innerhalb einer Stunde post applicationem. Die choleretische Wirkung nahm dann langsam bis zum Versuchsende ab.

Rowachol® zeigte hingegen eine langsamer einsetzende choleretische Wirkung, die ihr Maximum erst innerhalb der 3. Stunde nach Verabreichung der Prüfsubstanz erreichte und danach dann noch merklich weiter anhielt.

Dehydrocholsäure bewirkte direkt nach der Applikation einen sehr deutlichen Anstieg der Gallensäureausscheidung; das Maximum lag bereits nach 30 Minuten p.a., in der Folge fiel die Cholerese-Leistung leicht zurück.

cis-(3,3,5)-Trimethylcyclohexanol bewirkte in ähnlicher Weise einen mit der Applikation einsetzenden starken Anstieg der Cholerese, wobei die Wirkung aller anderen Prüfprodukte überschritten wurde. Das Maximum der Ausscheidung an Galleflüssigkeit wurde zwischen der 1. und 2. Stunde erreicht; die Ausscheidung war dann im weiteren Versuchsverlauf immer noch stark erhöht. Von besonderem Interesse ist die niedere Dosierung von cis-(3,3,5)-Trimethylcyclohexanol, die zur Erzielung des beschriebenen Effektes ausreichte.

Alle Prüfprodukte sind deutlich choleretisch wirksam. In den untersuchten Dosierungen zeigte cis-(3,3,5)-Trimethylcyclohexanol die deutlichste choleretische Wirkung, die sich auszeichnet
a) durch einen rasch einsetzenden Effekt,
b) durch einen anhaltenden Effekt und
c) durch eine niedere Dosierung.

Es ist hervorzuheben, dass cis-(3,3,5)-Trimethylcyclohexanol in der Dosis von 0,3 ml/kg eine bessere choleretische Wirkung zeigt als Rowachol® in der Dosis von 4,0 ml/kg. Der Gegenstand der Erfindung hat gegenüber Rowachol®, das ein aus 6 Terpenen zusammengesetztes Gemisch darstellt, den Vorteil einer Einzelsubstanz. Von besonderem Interesse ist dabei, dass die Toxizität von cis-(3,3,5)-Trimethylcyclohexanol nicht in entsprechendem Ausmass höher liegt.

Ferner wurden Toxizitätsprüfungen mit der Substanz an männlichen und weiblichen Ratten durchgeführt. Die Untersuchungen erfolgten in Anlehnung an «Appraisal of the safety of chemicals in Foods, Drugs and Cosmetics» by the Staff of the Division of Pharmacology, FDA, 1959. Die Testsubstanz wurde den Tieren leicht erwärmt und in flüssiger Konsistenz einmalig oral mittels starrer Schlundsonde appliziert. Unter diesen Versuchsbedingungen ergaben sich für die männlichen Ratten folgende Ergebnisse:

1. Die $LD_{50}$ für 24 Stunden konnte mit 3,40 ml/kg ermittelt werden und entsprach der 7-Tage-$LD_{50}$, da keine Spätmortalitäten auftraten.

2. Die Schwankungsbreite der akuten Toxizität war aufgrund des Neigungsfunktionsfaktors S gut abgrenzbar.

Die Ergebnisse der Versuche an weiblichen Ratten sind folgende:

1. Die $LD_{50}$ für 24 Stunden konnte mit 2,50 ml/kg ermittelt werden. Die 7-Tage-$LD_{50}$ betrug 2,40 ml/kg.

2. Die Schwankungsbreiten der akuten Toxizitäten waren aufgrund des Neigungsfunktionsfaktors S gut abgrenzbar.

Damit ergibt sich für die Substanz nach der Erfindung eine ausserordentlich gute Verträglichkeit. In Verbindung mit der zuvor beschriebenen choleretischen Wirkung stellt damit cis-(3,3,5)-Trimethylcyclohexanol eine Bereicherung der Medizin dar.

Es werden folgende Formulierungen empfohlen:

Der Wirkstoff wird mit einem magensaftresistenten Überzug versehen. Jede Kapsel enthält:

| | |
|---|---|
| cis-(3,3,5)-Trimethylcyclohexanol | 40,0 mg |
| Trägerstoff | 60,0 mg |
| gesamt | 100,0 mg |

Kapselgrösse: 32 minims rund
Gewicht der Kapselhülle: 75 mg
Zusammensetzung der Kapselhülle: Gelantine U.S.P., DAB 70%; Glycerin DAB 30%.

Die Gelantinehülle ist mit 0,3% p-Hydroxy-benzoesäure-natrium-äthylester (vgl. Extra Pharmacopoeia Martindale, Bd. I) und 0,15% p-Hydroxy-benzoesäure-natrium-propylester präpariert.

## Patentanspruch

Cis-(3,3,5)-Trimethylcyclohexanol zur Verwendung bei der Behandlung von Galleleiden und/oder bei der Erniedrigung der Cholesterinbildung in der Galle.

## Patent Claim

Use of cis-(3,3,5)-trimethylcyclohexanol for the treatment of biliary disorders and/or for reducing the cholesterol production in the bile.

## Revendication

Cis-(3,3,5)-triméthylcyclohexanol dans son utilisation pour le traitement des affections biliaires et/ou pour la diminution de la formation de cholestérol dans la bile.

GALLEVERSUCH
VOLUMEN

FIG.1

ZEIT IN MIN.

ML

△ III
⬠ II
⬦ I
× V
— K

0 002 803

GALLEVERSUCH
CHOLESTERIN

MG PROZENT

ZEIT IN MIN.

III
II
I
V
K

FIG.2

0 002 803

GALLEVERSUCH
TROCKENGEWICHT

FIG.3

Fig.4

K o— —o Kontrolle

1 ●—● cis-(3,3,5)-Trimethylcyclohexanol

2 ▲—▲ Dehydrocholsäure

3 △—△ Rowachol[R]

4 □—□ Bernsteinsäureester

Menge der ausgeschiedenen Gallenflüssigkeit
in mg/Fraktion